(19) Europäisches Patentamt
European Patent Office
Office européen des brevets

(11) Veröffentlichungsnummer: **0 251 013 B1**

(12) # EUROPÄISCHE PATENTSCHRIFT

(45) Veröffentlichungstag der Patentschrift: **21.08.91 Patentblatt 91/34**

(51) Int. Cl.[5]: **C07C 213/00**, C07C 215/76

(21) Anmeldenummer: **87108592.4**

(22) Anmeldetag: **15.06.87**

(54) **Fluorhaltige o-Aminophenole.**

(30) Priorität: **25.06.86 DE 3621215**

(43) Veröffentlichungstag der Anmeldung: **07.01.88 Patentblatt 88/01**

(45) Bekanntmachung des Hinweises auf die Patenterteilung: **21.08.91 Patentblatt 91/34**

(84) Benannte Vertragsstaaten: **CH DE FR GB LI**

(56) Entgegenhaltungen:
**EP-A- 0 134 400**
**EP-A- 0 166 287**
**WO-A-86/02646**
**GB-A- 2 163 154**

(56) Entgegenhaltungen:
**CHEMICAL ABSTRACTS, Band 55, Nr. 23, 13. November 1961, Spalte 23408d, Formel 1, Columbus, Ohio, USA; L.M. YAGUPOL SKII et al: "Synthesis of derivatives of phenyl trifluoromethyl ether";**
**CHEMICAL ABSTRACTS, Band 60, Nr. 2, 20. Januar 1964, Spalte 1732f, Formel 2, Columbus, Ohio, USA; L.M. YAGUPOL SKII et al.: "Synthesis of 5-methyl-6-triflouromethylbenzimidazole"**

(73) Patentinhaber: **BAYER AG**
**W-5090 Leverkusen 1 Bayerwerk (DE)**

(72) Erfinder: **Kysela, Ernst, Dr.**
**Virchowstrasse 14**
**W-5060 Bergisch Gladbach (DE)**
Erfinder: **Baasner, Bernd, Dr.**
**Hamberger Strasse 27d**
**W-5090 Leverkusen 3 (DE)**

**EP 0 251 013 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Es wurden neue fluorhaltige o-Aminophenole der Formel (I) gefunden

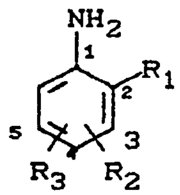

(I),

in der

$R_1$ für Hydroxyl,

$R_2$ für Methyl, Trifluormethoxy, 1,1,2,2-Tetrafluorethoxy oder Chlor und

$R_3$ für Wasserstoff, Trifluormethyl, Methoxy, Trifluormethoxy, 1,1,2,2-Tetrafluorethoxy oder 1,1,2-Trifluor-2-chlorethoxy oder

$R_2$ und $R_3$ gemeinsam für eine $-O-CF_2-O-CF_2$-Brücke stehen.

Vorzugsweise steht in Formel (I) $R_2$ in 3- oder 4-Position und $R_3$ in 5-Position.

Von den Verbindungen der Formel (I) mit $R_1$ = OH sind diejenigen bevorzugt, bei denen $R_2$ = 4 $OCF_3$ und $R_3$ = 5 $OCF_3$, $R_2$ = 4 $CF_3$ und $R_3$ = 5 $OCF_2CF_2H$, $R_2$ = 4 $OCF_2CF_2H$ und $R_3$ = H, $R_2$ = 4 Cl und $R_3$ = 5$CF_3$, $R_2$ = 4 $OCF_3$ und $R_3$ = H und $R_2$ und $R_3$ gemeinsam eine $-O-CF_2-O-CF_2$-Brücke, die in 3- und 4-Position angebunden ist, sind.

Die neuen Verbindungen können auf an sich bekannte Weise hergestellt werden (siehe L.M. Jagupolskij et al., Zh. obsh. Khim. 33, 3031-5 (1963), Houben-Weyl, Band X/1, S.559 (1971) und Band X1/1, S.472-3 (1957)).

Verbindungen der Formel (I) mit $R_1$ = OH kann man beispielsweise herstellen, indem man Verbindungen der Formel (III)

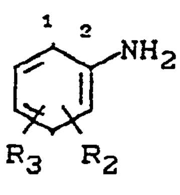

(III),

in der

$R_2$ und $R_3$ die bei Formel (I) angegebene Bedeutung haben, zunächst diazotiert und anschließend in Gegenwart von Schwefelsäure verkocht, wobei die $NH_2$- in eine OH-Gruppe überführt wird, dann nitriert und so eine $NO_2$-Gruppe in 1-Stellung einführt und diese schließlich zur Aminogruppe reduziert.

Die für das aufgezeigte Verfahren zur Herstellung der erfindungsgemäßen Verbindungen der Formel (I) benötigten Ausgangsprodukte der Formel (III) sind bekannt und auf bekannte Weise oder analog dazu herzustellen (siehe z.B. Zh. Org. Khim. 7 (1971) 7, 1469-73).

Die erfindungsgemäßen Verbindungen der Formel (I) können beispielsweise mit Trifluoressigsaure, gegebenenfalls in Gegenwart von hochkonzentrierter Salzsäure und/oder Verdünnungsmitteln umgesetzt und so 2-Trifluormethyl-benzimidazole der Formel (IV) erhalten werden, bei denen es sich um wertvolle Herbizide und Mikrobizide, insbesondere Fungizide, handelt.

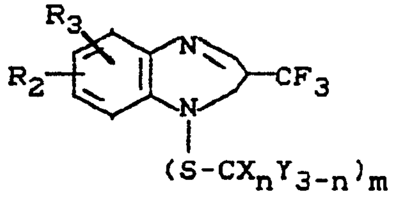

(IV)

In Formel (IV) haben $R_2$ und $R_3$ die bei Formel (I) angegebene Bedeutung, X und Y stehen unabhängig voneinander für gleiche oder verschiedene Halogenatome, n steht für Null, 1, 2 oder 3 und m steht für Null oder

1.

Zur Herstellung von Verbindungen der Formel (IV) mit m = 1 ist es erforderlich, nach der Bildung N-unsubstituierter 2-Trifluormethyl-benzimidazole diese noch mit einem entsprechenden Sulfensäurehalogenid umzusetzen.

Beispiel 1

a) 168 g (0,75 Mol) 4-Trifluormethyl-5-(1,1,2,2-tetrafluorethoxy)-anilin (Formel (III), $R_2$ = 4-Trifluormethyl, $R_3$ = 5-(1,1,2,2-Tetrafluorethoxy) wurden in 185 g konz. Salzsäure und 335 g Wasser mit 136 g 40 %iger Natriumnitrit-Lösung diazotiert und die entstandene Diazoniumsalzlösung in einer Mischung aus 290 g Wasser, 525 g konz. Schwefelsäure und 650 g Xylol verkocht. Es fielen 114 g 4-Trifluormethyl-5-(1,1,2,2-tetrafluorethoxy)-phenol mit einem Siedepunkt von 78° C bei 0,25 mbar an.

b) 112,5 g (0,5 Mol) 4-Trifluormethyl-5-(1,1,2,2-tetrafluorethoxy)-phenol wurden in 150 g 40 gew.-%iger Salpetersäure bei 20° C nitriert. Die wäßrige Aufarbeitung und anschließende Destillation ergaben 107,5 g 1-Nitro-2,-hydroxy-4-fluormethyl-5-(1,1,2,2-tetrafluorethoxy)-benzol (= 80 % der Theorie) mit einem Siedepunkt von 98° C bei 0,5 mbar.

c) Dieses wurde in 250 ml Methanol unter Zusatz von 10 g Raney-Nickel bei 40-50° C und 30-50 bar Wasserstoffdruck hydriert. Nach Abtrennung des Raney-Nickels und des Methanols wurden 68,5 g 1-Amino-2-hydroxy-4-trifluormethyl-5-(1,1,2,2-Tetrafluorethoxy)-benzol (Formel (I)), $R_1$ = OH, $R_2$ = 4 Trifluormethyl, $R_3$ = 5-(1,1,2,2-Tetrafluorethoxy) (= 72 % der Theorie) mit einem Schmelzpunkt von 104°C erhalten.

Beispiele 2 bis 5 :

Es wurde verfahren wie in Beispiel 1 beschrieben, jedoch wurde in Stufe a) jeweils eine andere Ausgangsverbindung der Formel (III) eingesetzt. Das jeweilige Ausgangsprodukt und das nach Stufe c) erhaltene Reaktionsprodukt, sowie Siede- und Schmelzpunkt des letzteren sind aus Tabelle 1 ersichtlich.

Tabelle 1

| Beispiel Nr. | Ausgangsprodukt der Formel (II) und Reaktionsprodukt der Formel (I) | Siedepunkt (°C/mbar) | Schmelzpunkt (°C) |
|---|---|---|---|
| 2 | $R_2$ = 4 $OCF_3$, $R_3$ = 5 $OCF_3$ | 120-2/8 | - |
| 3 | $R_2$ = 4 $OCF_2CF_2H$, $R_3$ = H | 142/6 | 103 |
| 4 | $R_2$ = 4 Cl, $R_3$ = 5 $CF_3$ | | 68-70 |
| 5 | $R_2$ = 4 $OCF_3$, $R_3$ = H | | 98 |

Beispiel 6

zu 21,05 g (0,1 Mol) 2-Amino-4-chlor-5-trifluormethylanilin (hergestellt nach Beispiel 4) wurden in 20 min 45 g (0,39 Mol) Trifluoressigsäure getropft. Die Temperatur stieg von 20° C auf etwa 60° C an, es wurde nicht gekühlt. Anschließend wurde in 30 min auf 130° C (Badtemperatur) aufgeheizt und 2 h bei dieser Temperatur nachgerührt. Nach Erkalten wurde das Gemisch durch Zutropfen von 120 ml 10%iger Natronlauge alkalisch gestellt und der ausgefallene Feststoff abgesaugt, mit Wasser nachgewaschen und getrocknet.

Ausbeute : 22,8 g (78,9 % der Theorie) 2,5-Bis(trifluormethyl)-6-chlorbenzimidazol (Formel (IV), $R_2$=Chlor, $R_3$ = Trifluormethyl, m = Null)
Schmelzpunkt : 185 - 187°C.

Beispiel 7

Zu 28,85 g (0,1 Mol) 2,5-Bis(trifluormethyl)-6-chlorbenzimidazol (hergestellt gemäß Beispiel 6) und 10,1 g (0,1Mol) Triethylamin in 120 ml Dichlormethan wurden zwischen 20° C und 30° C innerhalb einer Stunde 16,9 g (0,1 Mol) Dichlorfluormethylsulfenylchlorid, gelöst in 120 ml Dichlormethan, zugetropft. Es wurde 2 h bei gleicher Temperatur nachgerührt, das ausgefallene Triethylamin-Hydrochlorid abgesaugt und mit Dichlormethan

nachgewaschen. Das mit der Waschphase vereinigte Filtrat wurde zweimal mit Wasser gewaschen. Nach dem Trocknen ($MgSO_4$) wurden die flüchtigen Bestandteile im Wasserstrahlvakuum abdestilliert. Der ölige Rückstand wurde über eine kurze Kieselgelsäule chromatografisch filtriert (Fließmittel :Toluol), Nach Abziehen des Laufmittels erhielt man 18,1 g (42,9 % der Theorie) 1-Dichlorfluormethylsulfenyl-2,5-bis(trifluormethyl)-6-chlorbenzimidazol (Formel (IV), $R_2$ = Chlor, $R_3$-Trifluormethyl, m = 1, X = Chlor, Y = Fluor, n = 2) als gelbes Öl mit einer gaschromatografisch bestimmten Reinheit von 98,9% und einem Brechnungsindex $n_D^{20}$ von 1,5165.

Beispiel 8

Pre-emergence-Test

Lösungsmittel : 5 Gewichtsteile Aceton

Emulgator : 1 Gewichtsteil Alkylarylpolyglykolether

Zur Herstellung einer zweckmäßigen Wirkstoffzubereitung vermischte man 1 Gewichtsteil Wirkstoff mit der angegebenen Menge Lösungsmittel, gab die angegebene Menge Emulgator zu und verdünnte das Konzentrat mit Wasser auf die gewünschte Konzentration.

Samen der Testpflanzen wurden in normalen Boden ausgesät und nach 24 Stunden mit der Wirkstoffzubereitung begossen. Dabei wurde die Wassermenge pro Flächeneinheit zweckmäßigerweise konstant gehalten. Die Wirkstoffkonzentration in der Zubereitung spielte keine Rolle, entscheidend war nur die Aufwandmenge des Wirkstoffs pro Flächeneinheit. Nach drei Wochen wurde der Schädigungsgrad der Pflanzen bonitiert in % Schädigung im Vergleich zur Entwicklung der unbehandelten Kontrolle. Es bedeuten :

0 % = keine Wirkung (wie unbehandelte Kontrolle)

100 % = totale Vernichtung

Ergebnisse siehe Tabelle 2.

Tabelle 2: Pre-emergence-Test / Gewächshaus

| Wirkstoff | Wirkstoff-aufwand kg/ha | Baum-wolle | Weizen | Cheno-podium | Datura | Matri-caria | Portu-lak | Stellaria | Cynodon |
|---|---|---|---|---|---|---|---|---|---|
| 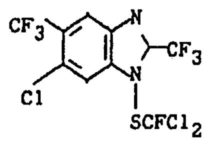 | 1,0 | 0 | 0 | 100 | 80 | 80 | 100 | 80 | 100 |

(gemäß Beispiel 7 erhalten)

Beispiel 9

Post-emergence-Test

Lösungsmittel : 5 Gewichtsteile Aceton

Emulgator : 1 Gewichtsteil Alkylarylpolyglykolether

Zur Herstellung einer zweckmäßigen Wirkstoffzubereitung vermischte man 1 Gewichtsteil Wirkstoff mit der angegebenen Menge Lösungsmittel, gab die angegebene Menge Emulgator zu und verdünnte das Konzentrat mit Wasser auf die gewünschte Konzentration.

Mit der Wirkstoffzubereitung spritzte man Testpflanzen, welche eine Höhe von 5 - 15 cm hatten so, daß die jeweils gewünschten Wirkstoffmengen pro Flächeneinheit ausgebracht wurden. Die Konzentration der Spritzbrühe wurde so gewählt, daß in 2000 l Wasser/ha die jeweils gewünschten Wirkstoffmengen ausgebracht wurden. Nach drei Wochen wurde der Schädigungsgrad der Pflanzen bonitiert in %. Schädigung im Vergleich zur Entwicklung der unbehandelten Kontrolle.

Es bedeuten :

0 % = keine Wirkung (wie unbehandelte Kontrolle)

100 % = totale Vernichtung

Ergebnisse siehe Tabelle 3.

Tabelle 3: Post-emergence-Test / Gewächshaus

| Wirkstoff | Wirkstoff-aufwand kg/ha | Baum-wolle | Mais | Cheno-podium | Sina-pis | Solanum | Echino-chloa | Poa |
|---|---|---|---|---|---|---|---|---|
| 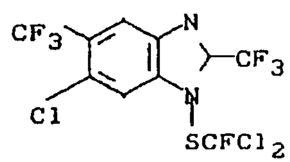 | 0,25 | 20 | 50 | 100 | 100 | 100 | 80 | 100 |

(gemäß Beispiel 7 erhalten)

## Patentansprüche

1. Fluorhaltige o-Aminophenole der Formel (I)

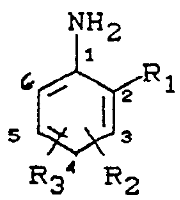

(I),

in der
$R_1$ für Hydroxyl,
$R_2$ für Methyl, Trifluormethoxy, 1,1,2,2-Tetrafluorethoxy oder Chlor und
$R_3$ für Wasserstoff, Trifluormethyl, Methoxy, Trifluormethoxy, 1,1,2,2-Tetrafluorethoxy oder 1,1,2-Trifluor-2-chlorethoxy oder
$R_2$ und $R_3$ gemeinsam für eine $-O-CF_2-O-CF_2-$Brücke stehen.

2. Fluorhaltige o-Aminophenole nach Anspruch 1, dadurch gekennzeichnet, daß sich $R_2$ in 3- oder 4-Position und $R_3$ in 5-Position befindet.

3. Fluorhaltige o-Aminophenole gemäß Ansprüchen 1 und 2, dadurch gekennzeichnet, daß $R_1$ für OH und $R_2$ für $4-OCF_3$ und $R_3$ für $5-OCF_3$, $R_2$ für $4-CF_3$ und $R_3$ für $5-OCF_2CF_2H$, $R_2$ für $4-OCF_2CF_2H$ und $R_3$ für H, $R_2$ für 4-Cl und $R_3$ für $5-CF_3$, $R_2$ für $4-OCF_3$ und $R_3$ für H oder $R_2$ und $R_3$ gemeinsam für eine $-O-CF_2-O-CF_2-$Brücke, die in 3-und 4-Position angebunden ist, steht.

4. Verfahren zur Herstellung von Verbindungen der Formel (I)

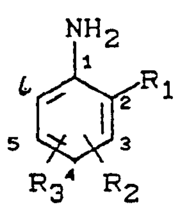

(I),

in der
$R_1$ für Hydroxyl,
$R_2$ für Methyl, Trifluormethoxy, 1,1,2,2-Tetrafluorethoxy oder Chlor und
$R_3$ für Wasserstoff, Trifluormethyl, Methoxy, Trifluormethoxy, 1,1,2,2-Tetrafluorethoxy oder 1,1,2-Trifluor-2-chlorethoxy oder $R_2$ und $R_3$ gemeinsam für eine $-O-CF_2-O-CF_2-$Brücke stehen,
dadurch gekennzeichnet, daß man Verbindungen der Formel (III)

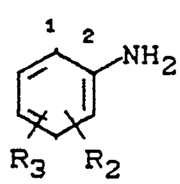

(III),

in der
$R_2$ und $R_3$ die bei Formel (I) angegebene Bedeutung haben, zunächst diazotiert und anschließend in Gegenwart von Schwefelsäure verkocht, wobei die $NH_2$-in eine OH-Gruppe überführt wird, dann nitriert und so eine $NO_2$-Gruppe in 1-Stellung einführt und diese schließlich zur Aminogruppe reduziert.

**Claims**

1. Fluorine-containing o-aminophenols of the formula (I)

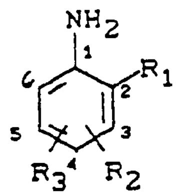

in which
$R_1$ represents hydroxyl,
$R_2$ represents methyl, trifluoromethoxy, 1,1,2,2-tetrafluoroethoxy or chlorine and
$R_3$ represents hydrogen, trifluoromethyl, methoxy, trifluoromethoxy, 1,1,2,2-tetrafluoroethoxy or 1,1,2-trifluoro-2-chloroethoxy, or
$R_2$ and $R_3$ together represent an $-O-CF_2-O-CF_2-$ bridge.

2. Fluorine-containing o-aminophenols according to Claim 1, characterised in that $R_2$ is in the 3- or 4-position and $R_3$ is in the 5-position.

3. Fluorine-containing o-aminophenols according to Claims 1 and 2, characterised in that $R_1$ represents OH and $R_2$ represents $4-OCF_3$ and $R_3$ represents $5-OCF_3$, $R_2$ represents $4-CF_3$ and $R_3$ represents $5-OCF_2CF_2H$, $R_2$ represents $4-OCF_2CF_2H$ and $R_3$ represents H, $R_2$ represents 4-Cl and $R_3$ represents $5-CF_3$, $R_2$ represents $4-OCF_3$ and $R_3$ represents H, or $R_2$ and $R_3$ together represent an $-O-CF_2-O-CF_2-$ bridge linked in the 3-and 4-position.

4. Process for the preparation of compounds of the formula (I)

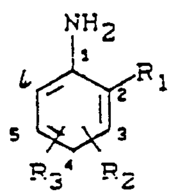

in which
$R_1$ represents hydroxyl,
$R_2$ represents methyl, trifluoromethoxy, 1,1,2,2-tetrafluoroethoxy or chlorine and
$R_3$ represents hydrogen, trifluoromethyl, methoxy, trifluoromethoxy, 1,1,2,2-tetrafluoroethoxy or 1,1,2-trifluoro-2-chloroethoxy, or
$R_2$ and $R_3$ together represent an $-O-CF_2-O-CF_2-$ bridge, characterised in that compounds of the formula (III)

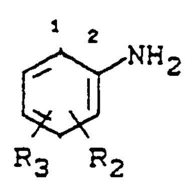

in which
$R_2$ and $R_3$ have the meaning given in the case of formula (I),
are first diazotised, the diazotisation mixture is then decomposed by boiling in the presence of sulphuric acid, the $NH_2$ group being converted into an OH group, the product is then nitrated and an $NO_2$ group is thus introduced into the 1-position, and, finally, this is reduced to the amino group.

**Revendications**

1. o-aminophénols fluorés de formule I :

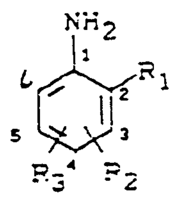

(I),

dans laquelle

$R_1$ représente un groupe hydroxy,

$R_2$ représente un groupe méthyle, trifluorométhoxy, 1,1,2,2-tétrafluoréthoxy ou le chlore et

$R_3$ représente l'hydrogène, un groupe trifluorométhyle, méthoxy, trifluorométhoxy, 1,1,2,2-tétrafluoréthoxy ou 1,1,2-trifluoro-2-chloréthoxy, ou bien

$R_2$ et $R_3$ forment ensemble un pont $-O-CF_2-O-CF_2-$.

2. o-aminophénols fluorés selon la revendication 1, caractérisés en ce que $R_2$ est en position 3 ou 4 et $R_3$ en position 5.

3. o-aminophénols fluorés selon les revendications 1 et 2, caractérisés en ce que $R_1$ représente OH et $R_2$ représente 4-$OCF_3$ et $R_3$ représente 5-$OCF_3$, $R_2$ représente 4-$CF_3$ et $R_3$ représente 5-$OCF_2CF_2H$, $R_2$ représente 4-$OCF_2CF_2H$ et $R_3$ représente H, $R_2$ représente 4-Cl et $R_3$ représente 5-$CF_3$, $R_2$ représente 4-$OCF_3$ et $R_3$ représente H ou bien $R_2$ et $R_3$ forment ensemble un pont $-O-CF_2-O-CF_2-$ relié dans les positions 3 et 4.

4. Procédé de préparation des composés de formule I :

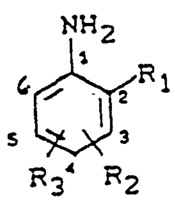

(I),

dans laquelle

$R_1$ représente un groupe hydroxy,

$R_2$ représente un groupe méthyle, trifluorométhoxy, 1,1,2,2-tétrafluoréthoxy ou le chlore et

$R_3$ représente l'hydrogène, un groupe trifluorométhyle, méthoxy, trifluorométhoxy, 1,1,2,2-tétrafluoréthoxy ou 1,1,2-trifluoro-2-chloréthoxy, ou bien

$R_2$ et $R_3$ forment ensemble un pont $-O-CF_2-O-CF_2$, caractérisé en ce que l'on diazote des composés de formule III :

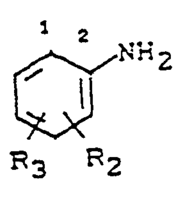

(III),

dans laquelle

$R_2$ et $R_3$ ont les significations indiquées en référence à la formule I, puis on soumet à ébullition en présence d'acide sulfurique, ce qui provoque la conversion du groupe $NH_2$ en un groupe OH, on nitre ensuite, introduisant ainsi un groupe $NO_2$ en position 1 et finalement on réduit ce groupe nitro en groupe amino.